# EUROPEAN PATENT SPECIFICATION

(11) **EP 2 687 156 B1**
(45) Date of publication and mention of the grant of the patent: **20.03.2019**
(21) Application number: 13176592.7
(22) Date of filing: 16.07.2013
(51) Int. Cl.: A61B 5/08, A61B 5/097, A61M 16/06

(54) **Gas sensor device**
Gassensorvorrichtung
Dispositif capteur à gaz

(30) Priority: 18.07.2012 GB 201212736; 11.12.2012 GB 201222281
(43) Date of publication of application: 22.01.2014
(73) Proprietor: Bedfont Scientific Limited, Maidstone, Kent ME17 1JA (GB)
(72) Inventor: Smith, Trevor, Maidstone, Kent ME17 1JA (GB); Smith, Jason, Kent, GB ME7 2QD (GB); Logan-Sinclair, Ron, Kent, GB ME2 3DD (GB)
(74) Representative: EIP

(56) References cited:
- EP-A1- 2 098 166
- US-A- 5 749 368
- US-B1- 6 484 724

## Description

The present invention relates to gas sensing devices and to mouthpieces therefor. More particularly it relates to gas sensor devices for detecting gases in exhaled air, and to improved mouthpieces and interfaces for use with such devices. The invention also provides methods for using such devices and kits.

Breath analysis, i.e. the analysis of the components of exhaled air, is emerging as a powerful diagnostic and prognostic tool in healthcare. For example, the detection of exhaled nitric oxide (ENO) is used for the identification of inflammation in the lungs and airways, e.g. for the diagnosis of asthma and other inflammatory conditions. Detecting hydrogen in breath can be used to detect or monitor lactose intolerance and carbohydrate maladsorption. Ammonia levels can be determined to investigate *H. pylori* activity in the stomach.

There are now a substantial number of devices available for conducting breath analysis. Much innovation has occurred in the area of sensor technology to allow faster and more accurate detection of target gases, and there has also been development in the clinical methodology techniques used in taking samples.

There has also been some development in respect of mouthpieces for ENO sampling, where it is important that a back pressure is applied and that the exhalation rate is maintained within specific boundaries. However, the mouthpieces used in the other areas of breath analysis, where carefully controlled rates of exhalation and application of back pressure is not of paramount importance, have not been developed.

For typical breath detectors, such as carbon monoxide and hydrogen detectors, mouthpieces comprise relatively wide-bore (i.e. 22 mm or higher) cardboard tubes which typically have a circular or elliptical cross section. These mouthpieces are disposable, typically with a new mouthpiece being used for each subject. These are essentially the same mouthpieces used for spirometry devices, and indeed spirometry mouthpieces were simply adopted for breath analysis devices when they began to emerge. There has been no move to improve these mouthpieces as they have been considered to be the perfectly optimal solution. The present inventors have, however, identified significant drawbacks associated with the use of such mouthpieces. For example, although light in weight, conventional mouthpieces are bulky and take up a significant volume when packaged - this has implications in terms of shipping and storage of mouthpieces, particularly as they are often required in large numbers. The wide nature of the mouthpieces can cause problems in that a subject has difficulty in controlling their exhalation rate through the mouthpiece; the exhalation rate subject through the mouthpiece can therefore be too rapid, meaning that the user runs out of breath before a sample is taken or that samples are taken incorrectly, e.g. due to the sensor not having enough sample to analyse.

Similar problems have been identified in document EP 2 098 166 A1, where an adaptor is provided for use with collapsible mouthpieces.

Document US 6 484 724 B1 discloses a breathing device adaptor for connection with different sized tubes and/or connectors.

The present invention is defined in claim 1, which provides improved mouthpiece systems for use in breath analysis.

The present invention provides an interface for the attachment of a mouthpiece to a gas sensor device, the interface comprising a first mouthpiece engaging means for engaging a first type of mouthpiece, and a second mouthpiece engaging means for engaging a second type of mouthpiece, the interface comprising an inlet positioned such that in use it is in fluid communication with the lumen of a mouthpiece mounted on either the first or the second mouthpiece engaging means.

Suitably the first mouthpiece engaging means is adapted to engage a larger mouthpiece than the second mouthpiece engaging means. This allows wide-bore mouthpieces (e.g. conventional mouthpieces) or narrow-bore mouthpieces to be connected to the same breath sensor device.

In an embodiment not according to the invention, the first and/or second mouthpiece engaging means suitably comprises a projection adapted to engage with the mouthpiece. The projection is preferably a flange. Conveniently both the first and second mouthpiece engaging means comprise flanges. A mouthpiece can be mounted over a suitably shaped flange, e.g. a tubular mouthpiece can be pushed over the flange. Alternatively, a mouthpiece can be mounted inside a flange, i.e. within a lumen defined by the flange. The flange can be continuous or discontinuous, provided that it provides sufficient support to mount a mouthpiece thereon.

Alternatively, the first and/or second mouthpiece engaging means can comprise a recess, e.g. a groove, adapted to engage with the mouthpiece. A mouthpiece can be mounted by inserting it into a suitably shaped recess.

In the present invention, the first and second mouthpiece engaging means are defined by a flange, the outside surface of which defines the first mouthpiece engaging means and the inner surface of which defines the second mouthpiece engaging means. Thus, the first mouthpiece engaging means is provided by a projection, i.e. the flange, and the second mouthpiece engaging means is defined by a recess, i.e. the lumen of said flange. This provides a significant advantage in that no trough is present between two flanges, and thus avoids the risk of condensation or the like being forming or being retained in the trough, as discussed further below.

In the present invention, the first and/or second mouthpiece engaging means are tapered such that a mouthpiece engages more tightly as it is pushed further onto or into the engaging means. Where the mouthpiece engaging means is a flange over which a mouthpiece is mounted, the flange tapers outwards in the proximal direction (i.e. towards the sensor device) and thus a mouthpiece engages more tightly with the inside of the mouthpiece as it is pushed further onto the engaging means. Where the mouthpiece engaging means is a recess into which the mouthpiece is mounted (e.g. the lumen defined by a flange), the recess tapers inwardly so it engages more tightly with the outside of a mouthpiece as it is pushed further into the engaging means.

The first and/or second mouthpiece engaging means can suitably comprise a shoulder adapted to abut against the leading edge of a mouthpiece when it is located in the correct position on the mouthpiece engaging means.

Preferably the first and/or second mouthpiece engaging means define a circle, ellipse, oval or polygon. It will be apparent that a range of mouthpiece shapes could be used, provided that they allow a user to form a relatively airtight seal with their lips. In a preferred embodiment the first mouthpiece engaging means defines an ellipse and the second mouthpiece engaging means defines a circle. Thus a particularly preferred embodiment comprises an elliptical flange for mounting a wide-bore mouthpiece and a circular flange or recess for mounting a narrow-bore mouthpiece.

It should be noted that in general the various terms for shapes are not used in their strict geometric sense and are intended to encompass shapes which deviate to some extent from strict mathematical rules. For example, the term 'ellipse' is intended to cover true ellipses but also other generally flattened circles or rounded lozenge shapes.

Suitably the second mouthpiece engaging means is located within the ambit of the first mouthpiece engaging means. The first and second mouthpiece engaging means can be concentric, for example.

The second mouthpiece engaging means is preferably adapted to engage a mouthpiece having a lumen with a cross-sectional area of 180 mm² or less, more preferably 80 mm² or less, optionally 50 mm² or less. These areas correlate with circular tubes of approximate diameter 15 mm, 10mm and 8mm, respectively. However, it should be noted that the mouthpiece need not be circular, but could also be elliptical, oval or polygonal, as discussed above. For non-circular cross-section mouthpieces, one can think in terms of mouthpieces having a lumen with a major width dimension of 15 mm or less, preferably 10 mm or less. By 'major width dimension' is meant the width of the lumen of the mouthpiece when measured in its widest dimension, e.g. the major axis of an ellipse. Furthermore it should be noted that the mouthpiece can have a constant cross section along its length, or it could have a cross-section that varies along its length in terms of shape or area. The relevant measurement is typically taken at the narrowest point along the length of the mouthpiece, as it is the narrowest point which will dictate the resistance to flow of exhaled air along the mouthpiece. Such mouthpieces will be referred to at points below as 'narrow-bore' mouthpieces.

The first mouthpiece engaging means is suitably adapted to engage a mouthpiece having a lumen with a cross-sectional area of 300 mm² or more, more preferably 380 mm² or more, optionally 700 mm² or more. These areas correlate with circular tubes of approximate diameter 20 mm, 22 mm and 30 mm, respectively. The above discussion with respect to the various shapes of cross-sections of the second mouthpiece applies in the same way in respect of the first mouthpiece. Such mouthpieces will be referred to at points below as 'wide-bore' mouthpieces.

Preferably the inlet through which the exhaled air passes into the breath sensor device is positioned within the ambit of the second mouthpiece engaging means. Typically the inlet has a similar area to the cross-sectional area of the mouthpiece, although it could be narrower.

The present invention provides a gas sensor device comprising an interface as set out above. The gas sensor device is a CO or H₂ sensor device. However, in a device not according to the invention, it could be any other type of gas sensor device which is used to measure a property of exhaled air.

The interface may comprise fixing means to fix the interface to a gas sensor device. For example the fixing means could comprise clip elements, holes for screws to engage with, or the like. The interface may alternatively be integral with a gas sensor device, e.g. formed as part of the casing of the device.

In a further aspect the present invention also provides the gas sensor device comprising a mouthpiece attached to the first or second mouthpiece engaging means. In a preferred embodiment there is provided such a gas sensor device comprising a mouthpiece having a cross-sectional area of 180 mm² or less, more preferably 80 mm² or less, optionally 50 mm² or less. Preferably the mouthpiece has a substantially circular cross-section and has a diameter of 10 mm or less attached to the second mouthpiece engaging means. Suitably the mouthpiece is from 70 mm to 130 mm long, preferably from 80 mm to 120 mm long, and more preferably from 90 mm to 110 mm long. Suitably the mouthpiece has a wall thickness of from 0.2 mm to 0.8 mm, preferably from 0.3 mm to 0.7 mm, and more preferably 0.4 mm to 0.6 mm.

The mouthpiece suitably comprises a marker, the marker indicating the correct distance for insertion of the mouthpiece into the mouth of a user. The marker can be a visible marker, or it could be a somatosensory (e.g. tactile) marker.

In a further aspect of the present invention there is provided a method of operating a gas sensor device comprising providing a gas sensor as set out above, providing a suitable mouthpiece and attaching the mouthpiece to the gas sensor device. The method may comprise causing a subject to exhale into the gas sensing device through the mouthpiece. The method may comprise causing a user to insert the mouthpiece into their mouth to a distance indicated by a marker provided on the mouthpiece.

In a further aspect the present invention provides a kit comprising a gas sensor device and/or an interface as set out above with one or more mouthpieces.

In a further aspect the present invention provides a carbon monoxide or hydrogen gas sensor device comprising a replaceable mouthpiece attached thereto (e.g. via an interface), the mouthpiece comprising a tube having a cross-sectional area of 180 mm² or less, more preferably 80 mm² or less, optionally 50 mm² or less.

It is not known in the art to use a narrow-bore mouthpiece with a carbon monoxide or hydrogen gas sensor device. Such a mouthpiece provides significant advantages when used with a carbon monoxide or hydrogen gas sensor device, as will be discussed further below.

Various properties of suitable narrow-bore mouthpieces are discussed above in relation to earlier aspects of the invention. Preferably the mouthpiece has a substantially constant cross-section, e.g. a circular or elliptical cross-section. Preferably the mouthpiece is from 70 mm to 130 mm long, preferably from 80 mm to 120 mm long, and more preferably from 90 mm to 110 mm long. Suitably the mouthpiece has a wall thickness of from 0.2 mm to 0.8 mm, preferably from 0.3 mm to 0.7 mm, and more preferably 0.4 mm to 0.6 mm. The mouthpiece suitably comprises a marker, the marker indicating the correct distance for insertion of the mouthpiece into the mouth of a user.

In a further aspect there is provided a method of operating a carbon monoxide or hydrogen gas sensor device comprising providing a carbon monoxide or hydrogen gas sensor device and attaching thereto a mouthpiece comprising a tube having a cross-sectional area of 180 mm² or less, more preferably 80 mm² or less, optionally 50 mm² or less. The method can comprise inserting the mouthpiece into a user's mouth, e.g. to a distance indicated by a marker provided on the mouthpiece. The method can comprise causing a subject to exhale into the gas sensor device.

In a further aspect there is provided a kit comprising a carbon monoxide or hydrogen gas sensor device and one or more mouthpieces comprising a tube having a cross-sectional area of 180 mm² or less, more preferably 80 mm² or less, optionally 50 mm² or less.

In a further aspect the present invention provides the use of a mouthpiece having a cross-sectional area of 180 mm² or less with a carbon monoxide or hydrogen gas sensor device.

Embodiments of the invention will now be described, by way of example only, with reference to the accompanying drawings in which:
- Fig. 1 shows line drawing side and front views an interface not according to the present invention;
- Fig. 2 shows a cross-sectional view of the same interface as Fig. 1;
- Fig. 3 shows a cross-sectional view of the second mouthpiece engaging means of the same interface;
- Fig. 4 shows side and end views of a mouthpiece;
- Fig. 5 shows line drawing side cross-section and isometric views of the interface with a mouthpiece attached to the second mouthpiece engaging means of the interface;
- Fig. 6 shows a mouthpiece packaged prior to use; and
- Fig. 7 shows cross-section and isometric views of an interface according to the present invention.

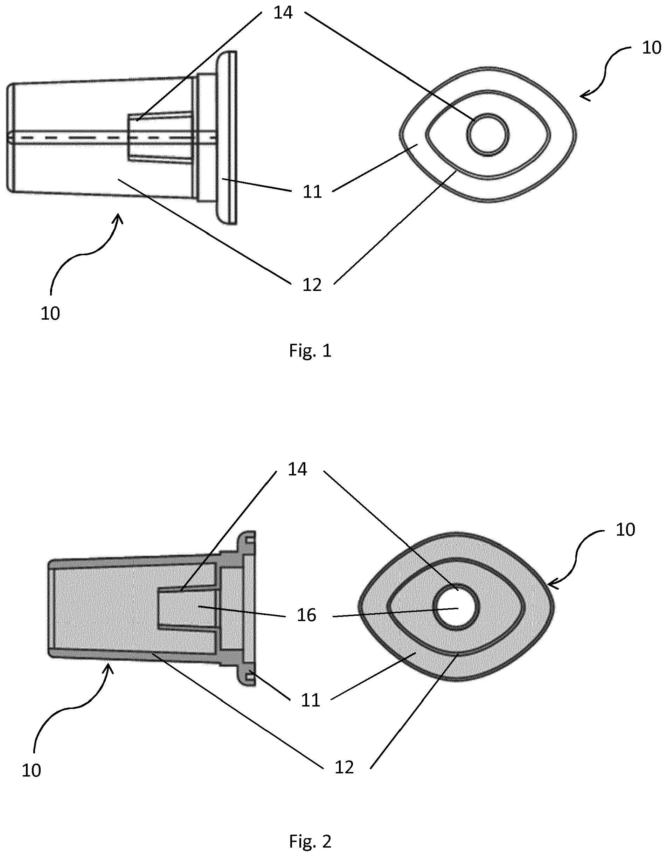

As shown in Figs 1 and 2, there is provided an interface 10, for a breath sensor device comprising a first mouthpiece engaging means 12 and a second mouthpiece engaging means 14. Inside the second mouthpiece engaging means there is an inlet 16 through which exhaled air passes from the lumen of the mouthpiece into the gas sensor device.

The first mouthpiece engaging means 12 comprises a substantially elliptical flange which projects from the base 11 of the interface 10. The flange tapers gently from the proximal end (i.e., the end nearest the gas sensor device) to the distal end such that the circumference of the first mouthpiece engaging means 12 decreases slightly as it extends away from the base 11. This facilitates initial attachment of a mouthpiece to the first mouthpiece engaging means, and allows for a suitably tight attachment as the mouthpiece is slid along the first mouthpiece engaging means. The first mouthpiece engaging means 12 is suitable for the attachment of a conventional mouthpiece in the form of a cardboard tube (e.g. of the type available from Bedfont Scientific Ltd, UK).

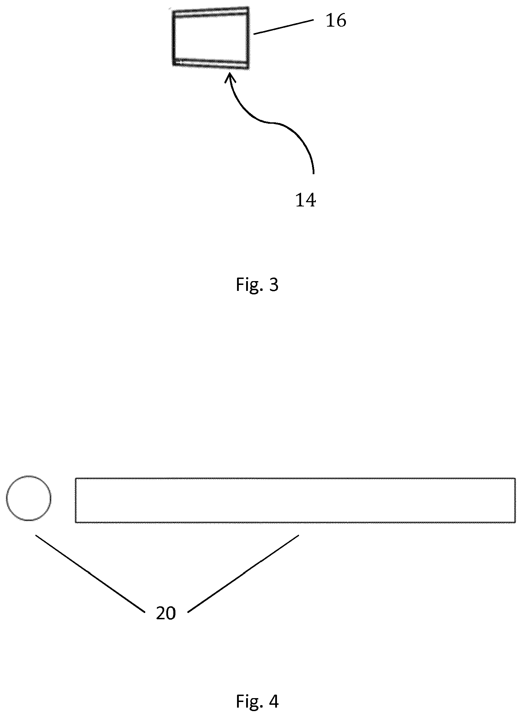

The second mouthpiece engaging means 14 comprises a substantially circular flange which extends from the base 11 of the interface 10, and which is located within the first mouthpiece engaging means 12. The second mouthpiece engaging means 14 is tapered in a similar manner to the first mouthpiece engaging means 12. The first and second mouthpiece engaging means 11,12 are concentric in the embodiment shown, though it is not necessary that they be so. Fig. 3 shows a more detailed view of the second mouthpiece engaging means, which includes an outer diameter which tapers from 10.1 mm at the proximal end to 9.9 mm at the distal end, and a constant wall thickness of 1.5mm. The second mouthpiece engaging means 12 is suitable for the attachment of a mouthpiece having a significantly smaller diameter than the conventional mouthpieces used in respect of breath sensor devices. The specific embodiment illustrated is suited for the attachment of a mouthpiece of generally circular cross-section, and having a diameter of 10 mm. The taper allows such a mouthpiece to be easily placed over the second mouthpiece engaging means, given the reduced diameter, and allows the mouthpiece to be fitted firmly and sufficiently airtight by sliding the mouthpiece further onto the second mouthpiece engaging means 14. Typically the material of the mouthpiece will be slightly resilient, allowing the mouthpiece to stretch slightly as is slides onto the second mouthpiece engaging means until it abuts against the base 11 of the interface 10. Thus a snug and sufficiently airtight seal can be easily achieved.

The interface is connected to a breath sensor device, e.g. a CO or H₂ sensor. The base 11 can be connected to the device by a clip mechanism and/or can be retained in position by fixing means such as screws or adhesive. In the embodiment shown the interface is connected to the device using screws.

Figure 4 shows a suitable mouthpiece 20 for attachment to the second mouthpiece engaging means 14. It is a circular cross-section tube having a substantially constant diameter of 8mm and a length of 100mm. The mouthpiece has a wall thickness of approximately 0.5 mm. Such a thickness has been found to provide a suitably strong tube which does not collapse when a user applies pressure to seal their lips around it, but which minimises use of material to avoid wastage and minimise cost. Suitably the tube is formed from a polymer, although other materials could be used provided they are compatible with the intended use, are non-toxic, and do adversely interact with the target gas. For example, a cardboard or paper tube could potentially be used. Suitable polymeric materials include polyurethane, polypropylene and polyethylene, but many other polymeric materials may be suitable.

The mouthpiece can comprise an antimicrobial agent. For example, the polymer may comprise an antimicrobial additive, e.g. triclosan which is a chlorinated aromatic compound with antibacterial, antifungal and antiviral properties (sold under several trade names including Microban, UltraFresh, Amicor, and BioFresh). Alternatively, silver compounds can be impregnated or combined with various materials to provide antimicrobial effects.

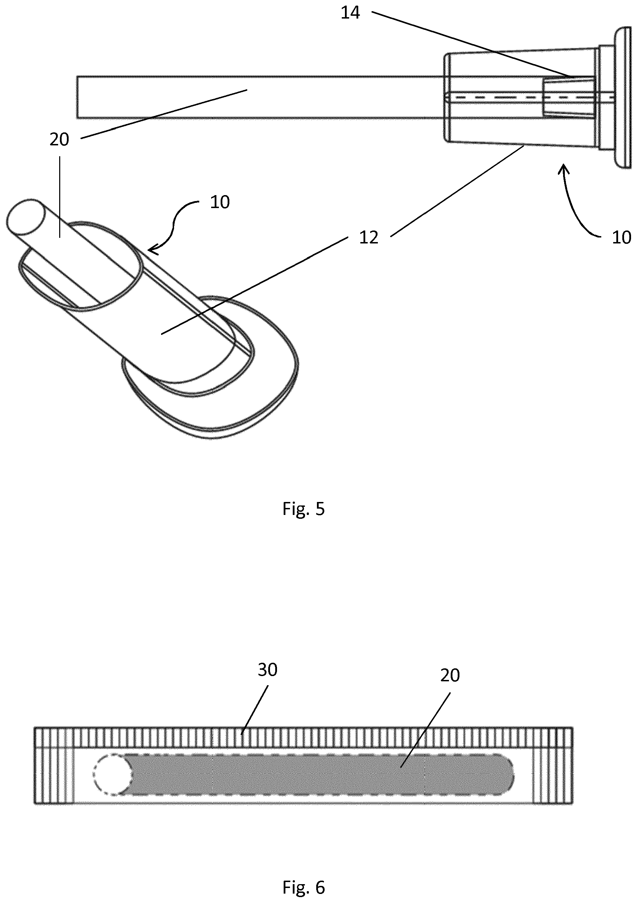

Fig. 5 shows the interface 10 with a mouthpiece 20 attached to the second mouthpiece engaging means 14.

As shown in Fig. 6 a mouthpiece 20 can be packaged in a wrapper 30, which allows the mouthpiece to be kept clean and optionally sterile prior to use. The wrapper 30 is formed from a sheet material, such as a polymer of paper. The wrapper is initially a flat rectangle, which is wrapped around the mouthpiece 20 and then the edges are sealed, e.g. using an adhesive or through thermal or ultrasonic welding; the preferred sealing method depends on, for example, the wrapper material. In Fig. 5 the sealed areas are shown with vertical hatching, and are 10 mm long at each end, and 5 mm wide down the side. The lumen in which the mouthpiece is held is approximately 120 mm long.

The present interface provides significant improvements over prior art interfaces for breath sensor devices, as will be discussed below. It can be used essentially any type of breath sensor device, but in preferred embodiments the sensor device is a carbon monoxide or hydrogen sensor device.

The ability to attach a narrow-bore mouthpiece provides several advantages. Narrow-bore mouthpieces make it easier for a user to exhale into the device at a desired flow rate. Conventional wide-bore mouthpieces provide minimal resistance to exhalation; indeed, that is an effect of their original purpose in spirometry and peak flow devices where any restriction to exhalation is undesirable. However, in breath sensor devices which detect concentrations of a constituent gas this feature is not advantageous, and indeed the present invention resides at least partially in the observation that it can be positively disadvantageous.

With a wide-bore mouthpiece it is all too easy for a subject to exhale too quickly and therefore run out of breath before a successful measurement has been taken. Even with modern sensors it still takes several seconds for a measurement to be accurately taken, and it is desirable to take the measurement during the middle of an exhalation. Where a sample is taken at the end phase of exhalation, which often occurs if a subject exhales too quickly initially, gas levels can be altered due to the comparatively high proportion of air from the residual volume of the lungs. This is obviously detrimental to the usefulness of the test because the concentration of target gas detected, though accurately measured, is not representative of typical tidal exhalation concentrations.

Furthermore, as many users are familiar with spirometry or peak flow type tests, where rapid exhalation is desirable, when confronted with a similar mouthpiece to that which is used in the test which is familiar to them, there can be a predisposition for such users to exhale rapidly. Although such a tendency can perhaps be countered by instructions being given to a subject doing the test by a clinician, this relies on the clinician giving correct instructions and, moreover, requires that a clinician is actually present, which adds to the cost of testing and may not be possible with home tests, for example. Written instructions are often not read and/or followed.

Spirometry mouthpieces are generally available in two forms, adult and paediatric. These forms are both typically circular, semi-rigid tubes, usually formed of cardboard, and the former has a diameter of approximately 30 mm and the latter of approximately 22 mm. Both of these mouthpieces are commonly used in gas sensor devices.

By using a narrow-bore mouthpiece instead, this provides a degree of resistance which tends to reduce the rate of exhalation. Providing a degree of exhalation resistance allows a subject to gauge how fast they are exhaling and adjust the rate accordingly. Where a wide mouthpiece is used and there is very little resistance to exhalation, it is extremely difficult to judge how fast one is exhaling. Furthermore, even absent a significant amount of resistance, it appears that a user is more easily able to control exhalation rate when using a narrow mouthpiece rather than a wide one. The reason for this is unclear, but perhaps is due to a user being accustomed to controlling the flow rate of exhaled air when exhaling through pursed lips, e.g. as used when whistling or when blowing normally. Additionally, because the mouthpiece is not of the type conventionally used in spirometry and peak flow testing, subjects familiar with such tests will not be predisposed to use the already similar, i.e. fast, exhalation style.

Further advantages can be provided by using a mouthpiece which is adapted to extend into the mouth beyond the teeth. For example, a mouthpiece which extends 4 cm or more into the mouth (i.e. this distance beyond the lips), can reach past the teeth of a subject into the oral cavity towards the isthmus faucium. Such a mouthpiece ensures that contamination by gases produced by dental microbes is kept to a minimum. The mouthpiece should not extend so far as to cause gagging, to impact on the throat, or to be occluded by tissues of the mouth or throat. Conveniently, a marker can be provided on a mouthpiece to provide guidance to a user as to how far the mouthpiece should be inserted into the mouth, e.g. a marker to show where a user's lips should be placed.

Using narrow-bore mouthpieces has the advantage of reducing the amount of material per mouthpiece and thus reducing costs and environmental impact. Furthermore it reduces the bulk of the packaged products considerably.

The interface described above provides the benefits described above by allowing convenient connection of a narrow-bore mouthpiece. However, it also allows a conventional wide-bore mouthpiece to be attached to the device. Thus dual utility is a distinct advantage in settings where both types of mouthpieces may be found, e.g. in medical facility where spirometry devices and associated mouthpieces are also present. A particular advantage of the interface is that even when a wide-bore mouthpiece is attached, the inlet is of restricted size compared with the diameter of the attached mouthpiece and therefore there is a degree of flow restriction provided. This therefore makes it easier for a subject to control exhalation rate.

It should be noted that the above described device and interface is a preferred embodiment not according to the invention, but various other embodiments within the scope of the invention are also envisaged. For example, carbon monoxide and hydrogen breath sensor devices are not known in the art in combination with a mouthpiece having a relatively small diameter. As mentioned in the introduction, CO and H₂ sensor devices are conventionally used in conjunction with a wide-bore mouthpiece, essentially as used in spirometry devices.

Modification of a CO or H₂ breath sensor device to comprise an interface adapted to engage a narrow-bore mouthpiece (e.g. having a diameter of 15 mm or less) allows the abovementioned advantages associated with narrow-bore mouthpieces to be provided to CO or H₂ sensing. This could be achieved by providing a multi mouthpiece interface as discussed above. Alternatively, in another aspect of the invention, this can be achieved by providing a single mouthpiece interface to the CO or H₂ breath sensor, the interface having a mouthpiece engaging means which is adapted to engage a narrow-bore mouthpiece.

Referring to a device comprising a dual mouthpiece interface, to use the device a user takes a narrow-bore mouthpiece and removes it from its wrapper. The mouthpiece is then pushed onto the second mouthpiece engaging means until it is securely mounted in position in the gas sensor device - this may be the point when the mouthpiece abuts against the base of the interface. The user then ensures the gas sensor device is operating and ready to receive a sample. The mouthpiece is then inserted into the mouth, preferably with the distal tip of the mouthpiece extending well past the teeth and into mouth cavity. The correct distance of insertion can be indicated by a mark provided on the mouthpiece, for example that the user aims to close their lips upon. Furthermore, the length of the mouthpiece can be selected such that over-insertion of the mouthpiece such that it touches the back of the throat or otherwise causes gagging is avoided. For example, the length of the mouthpiece can be selected such that, for a typical user, their lips will contact the interface or another part of the breath sensor device before the tip of the mouthpiece extends too far into the mouth, e.g. to contact the back of the mouth or throat. A length of 100mm has been found to work well. Once the lips have been sealed around the mouthpiece, the user exhales into the device to provide a sample. Exhalation is continued until the device indicates that the test has been conducted.

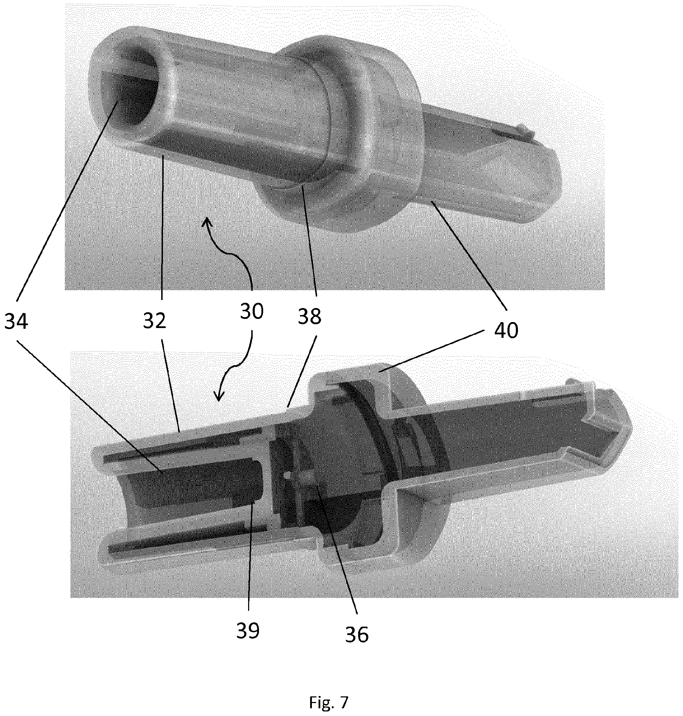

An embodiment according to the invention is shown in Fig 7. In this embodiment the interface 30 comprises a first mouthpiece engaging means 32 and a second mouthpiece engaging means 34. Within the ambit of the second mouthpiece engaging means there is an inlet 36 through which exhaled air passes from the lumen of the mouthpiece into the gas sensor device. The interface is shown connected to a housing 40 which is part of the gas sensor device.

In this embodiment the interface comprises a single flange, the outer surface of which defines the first mouthpiece engaging means 32, and the inner surface of which defines the second mouthpiece engaging means 34.

The first mouthpiece engaging means 32 is generally elliptical in section and tapers gently outwards in the proximal direction. Near the proximal end of the first mouthpiece engaging means there is a shallow annular shoulder 38 which projects outwards. A wide-bore mouthpiece can thus be slid onto the first mouthpiece engaging means, i.e. over the flange, the taper resulting in an increasingly tight fit as it is slid further on, until the mouthpiece abuts against shoulder 38 once it is correctly located.

The second mouthpiece engaging means 34 is generally circular in section and tapers gently inward in the proximal direction. Near the proximal end of the second mouthpiece engaging means there is a shallow annular shoulder 39 which projects outwards. A narrow-bore mouthpiece can thus be slid into the second mouthpiece engaging means, i.e. into the lumen defined by the flange, the taper resulting in an increasingly tight fit as it is slid further into the lumen, until the mouthpiece abuts against shoulder 39 once it is correctly located. The flange thus has a wall thickness which increases in the proximal direction which provides the taper to the first and second mouthpiece engaging means. In the embodiment shown in the figures the flange has a hollow wall structure, i.e. it has an inner and outer wall with a cavity therebetween, and there is an end wall portion which joins the inner and outer wall together at the distal end, thus forming a single hollow walled flange. However, it should be noted that the wall of the single flange need not have the hollow, double-walled structure described, e.g. it could be solid. The thickness of the wall of the flange generally corresponds to the difference between the diameters of the narrow- and wide-bore mouthpieces.

This embodiment has certain advantages over the embodiment described above. Where two separate flanges are provided there is a risk that condensation can form in the trough between the outer and inner flanges. Such condensation may not evaporate between uses of the device and thus there is a risk that droplets of condensation could run from the trough down the mouthpiece and contact a user. Furthermore, there is a risk that the trough could become unsanitary if not regularly cleaned, e.g. because the damp environment would be suitable for the growth of microbes or simply because dust and dirt could become trapped there. Any such problems could be avoided by regular cleaning and/or drying of the interface, but it would be preferably if the potential for the problem to arise were removed. The embodiment avoids the presence of such a trough by using a single flange with a comparatively thick wall, thus allowing the single flange to provide both the first and second mouthpiece engaging means via its inner and outer surfaces. The embodiment thus provides significant improvements in terms of avoiding cross infection and/or an unpleasant experience for a user.

Various modifications to the invention can be made without departing from the scope of the invention, which is defined in the appended claims. The flange of the mouthpiece engaging means described in the preferred embodiment above is a continuous elliptical or circular flange, but a discontinuous flange could be used. Additional retention means can be provided to hold the mouthpiece in position, e.g. high friction regions on the mouthpiece engaging means or a clip mechanism.

## Claims

1. A gas sensor device configured to detect CO or H₂ and comprising:
an interface (10) for the attachment of a mouthpiece (20) to the gas sensor device, the interface comprising:
a first mouthpiece engaging means (12) for engaging a first type of mouthpiece, and
a second mouthpiece engaging means (14) for engaging a second type of mouthpiece, the interface (10) comprising an inlet (16) positioned such that in use it is in fluid communication with the lumen of a mouthpiece mounted on either the first (12) or the second (14) mouthpiece engaging means,
wherein the first (12) and second (14) mouthpiece engaging means are defined by a flange,
the outside of the flange defining the first mouthpiece engaging means (12) and tapering outwards in a proximal direction, the proximal direction being towards a proximal end of the flange nearer the inlet than a distal end of the flange, and
the inside of the flange defining the second mouthpiece engaging means (14) and tapering inwards in the proximal direction.

2. The gas sensor device of claim 1 wherein the first mouthpiece engaging means (12) is adapted to engage a larger mouthpiece than the second mouthpiece engaging means (14).

3. The gas sensor device of any preceding claim, wherein the second mouthpiece engaging means (14) is adapted to engage a mouthpiece (20) having a lumen with a cross-sectional area of 180 mm² or less and wherein the first mouthpiece engaging means (12) is adapted to engage a mouthpiece having a lumen with a cross-sectional area of 300 mm2 or more.

4. The gas sensor device of any preceding claim wherein the first (12) and/or second (14) mouthpiece engaging means defines a circle or ellipse, preferably wherein both the first (12) and second (14) mouthpiece engaging means define a circle or ellipse, and yet more preferably wherein the first mouthpiece engaging means (12) defines an ellipse and the second mouthpiece engaging means (14) defines a circle.

5. The gas sensor device of any preceding claim wherein the second mouthpiece engaging means (14) is adapted to engage a mouthpiece (20) having a lumen with a cross-sectional area of 80 mm² or less, optionally 50 mm² or less, and/or wherein the first mouthpiece engaging means (12) is adapted to engage a mouthpiece having a lumen with a cross-sectional area of 380 mm² or more, optionally 700 mm² or more.

6. A gas sensor device comprising an interface (10) as defined in any one of claims 1 to 6, further comprising a mouthpiece attached to the first (12) or second (14) mouthpiece engaging means, and preferably comprising a mouthpiece (20) having a substantially circular cross-section and having a diameter of 10mm or less attached to the second mouthpiece engaging means (14).

7. The gas sensor device according to claim 6 which comprises a mouthpiece comprising a marker, the marker indicating the correct distance for insertion of the mouthpiece into the mouth of a user.

8. A method of operating the gas sensor device comprising the steps of:
(1) providing a gas sensor device according to any one of claims 1 to 7,
(2) providing a suitable mouthpiece (20), and
(3) attaching the mouthpiece to the gas sensor device, and preferably
(4) causing a subject to be tested to exhale into the gas sensing device through the mouthpiece (20), wherein the mouthpiece (20) preferably has a cross-sectional area of 180 mm² or less, more preferably 80 mm² or less, optionally 50 mm² or less.

9. A kit comprising a gas sensor device of any one of claims 1 to 7 with one or more mouthpieces (20).

## Patentansprüche

1. Gassensorvorrichtung, die eingerichtet ist, CO oder H₂ zu erfassen, umfassend:
eine Schnittstelle (10) zur Befestigung eines Mundstücks (20) an der Gassensorvorrichtung, wobei die Schnittstelle umfasst:
ein erstes Mundstückeingriffsmittel (12) zum Eingreifen in einen ersten Mundstücktyp; und
ein zweites Mundstückeingriffsmittel (14) zum Eingreifen in einen zweiten Mundstücktyp,
wobei die Schnittstelle (10) einen Einlass (16) umfasst, der so positioniert ist, dass er im Gebrauch in fluidischer Verbindung mit dem Volumen eines Mundstücks steht, das entweder auf das erste (12) oder zweite (14) Mundstückeingriffsmittel montiert ist,
wobei das erste (12) und zweite (14) Mundstückeingriffsmittel durch einen Flansch definiert sind, die Außenseite des Flansches das erste Mundstückeingriffsmittel (12) definiert und sich in einer proximalen Richtung nach außen verjüngt, wobei die proximale Richtung auf ein proximales Ende des Flansches gerichtet ist, das dem Einlass näher als ein distales Ende des Flansches ist, und
die Innenseite des Flansches das zweite Mundstückeingriffsmittel (14) definiert und sich in der proximalen Richtung nach innen verjüngt.

2. Gassensorvorrichtung nach Anspruch 1, wobei das erste Mundstückeingriffsmittel (12) angepasst ist, in ein größeres Mundstück als das zweite Mundstückeingriffsmittel (14) einzugreifen.

3. Gassensorvorrichtung nach einem der vorgehenden Ansprüche, wobei das zweite Mundstückeingriffsmittel (14) angepasst ist, in ein Mundstück (20) einzugreifen, das ein Volumen mit einer Querschnittsfläche von 180 mm² oder weniger aufweist, und wobei das erste Mundstückeingriffsmittel (12) angepasst ist, in ein Mundstück einzugreifen, das ein Volumen mit einer Querschnittsfläche von 300 mm² oder mehr aufweist.

4. Gassensorvorrichtung nach einem der vorhergehenden Ansprüche, wobei das erste (12) und/oder zweite (14) Mundstückeingriffsmittel einen Kreis oder eine Ellipse definiert, wobei vorzugsweise beide von den ersten (12) und zweiten (14) Mundstückeingriffsmitteln einen Kreis oder eine Ellipse definieren und wobei weiter bevorzugt das erste Mundstückeingriffsmittel (12) eine Ellipse und das zweite Mundstückeingriffsmittel (14) einen Kreis definiert.

5. Gassensorvorrichtung nach einem der vorhergehenden Ansprüche, wobei das zweite Mundstückeingriffsmittel (14) angepasst ist, in ein Mundstück (20) mit einem Volumen mit einer Querschnittsfläche von 80 mm² oder weniger, optional 50 mm² oder weniger, einzugreifen und/oder wobei das erste Mundstückeingriffsmittel (12) angepasst ist, in ein Mundstück mit einer Querschnittsfläche von 380 mm² oder mehr, optional 700 mm² oder mehr, einzugreifen.

6. Gassensorvorrichtung mit einer Schnittstelle (10), wie sie in einem der Ansprüche 1 bis 6 definiert ist, ferner umfassend ein Mundstück, das mit dem ersten (12) oder zweiten (14) Mundstückeingriffsmittel verbunden ist, und vorzugsweise ein Mundstück (20) umfasst, das im Wesentlichen einen kreisförmigen Querschnitt aufweist, der einen Durchmesser von 10 mm oder weniger aufweist, und mit dem zweiten Mundstückeingriffsmittel (14) verbunden ist.

7. Gassensorvorrichtung nach Anspruch 6, die ein Mundstück mit einer Markierung umfasst, wobei die Markierung den korrekten Abstand für das Einfügen des Mundstücks in den Mund eines Benutzers anzeigt.

8. Verfahren zum Betreiben einer Gassensorvorrichtung, umfassend die Schritte:
(1) Bereitstellen einer Gassensorvorrichtung nach einem der Ansprüche 1 bis 7,
(2) Bereitstellen eines passenden Mundstücks (20) und
(3) Verbinden des Mundstücks mit der Gassensorvorrichtung und vorzugsweise
(4) Veranlassen einer Testperson, in die Gassensorvorrichtung durch das Mundstück (20) auszuatmen, wobei das Mundstück (20) vorzugsweise eine Querschnittsfläche von 180 mm² oder weniger, mehr bevorzugt 80 mm² oder weniger, optional 50 mm² oder weniger, aufweist.

9. Satz umfassend eine Gassensorvorrichtung nach einem der Ansprüche 1 bis 7 mit einem oder mehreren Mundstücken (20).

## Revendications

1. Dispositif capteur de gaz configuré pour détecter du CO ou du H₂ et comprenant :
une interface (10) pour la fixation d'un embout buccal (20) au dispositif capteur de gaz, l'interface comprenant :
un premier moyen d'engagement d'embout buccal (12) pour s'engager avec un premier type d'embout buccal, et
un deuxième moyen d'engagement d'embout buccal (14) pour s'engager avec un deuxième type d'embout buccal, l'interface (10) comprenant une entrée (16) positionnée de sorte qu'elle soit, en utilisation, en communication fluidique avec la lumière d'un embout buccal monté soit sur le premier (12) ou sur le deuxième (14) moyen d'engagement d'embout buccal,
dans lequel les premier (12) et deuxième (14) moyens d'engagement d'embout buccal sont définis par une bride,
la partie externe de la bride définissant le premier moyen d'engagement d'embout buccal (12) et se rétrécissant vers l'extérieur dans une direction proximale, la direction proximale se trouvant vers une extrémité proximale de la bride plus proche de l'entrée que d'une extrémité distale de la bride, et
la partie interne de la bride définissant le deuxième moyen d'engagement d'embout buccal (14) et se rétrécissant vers l'intérieur dans la direction proximale.

2. Dispositif capteur de gaz de la revendication 1, dans lequel le premier moyen d'engagement d'embout buccal (12) est adapté pour s'engager avec un embout buccal plus grand que celui avec lequel s'engage le deuxième moyen d'engagement d'embout buccal (14).

3. Dispositif capteur de gaz de l'une des revendications précédentes, dans lequel le deuxième moyen d'engagement d'embout buccal (14) est adapté pour s'engager avec un embout buccal (20) ayant une lumière qui a une surface de section transversale inférieure ou égale à 180 mm² et dans lequel le premier moyen d'engagement d'embout buccal (12) est adapté pour s'engager avec un embout buccal ayant une lumière qui a une surface de section transversale supérieure ou égale à 300 mm².

4. Dispositif capteur de gaz de l'une des revendications précédentes, dans lequel le premier (12) et/ou le deuxième (14) moyen(s) d'engagement d'embout buccal définit/définissent un cercle ou une ellipse, dans lequel de préférence à la fois les premier (12) et deuxième moyens (14) d'engagement d'embout buccal définissent un cercle ou une ellipse, et dans lequel encore plus préférablement le premier moyen d'engagement d'embout buccal (12) définit une ellipse et le deuxième moyen d'engagement d'embout buccal (14) définit un cercle.

5. Dispositif capteur de gaz de l'une des revendications précédentes, dans lequel le deuxième moyen d'engagement d'embout buccal (14) est adapté pour s'engager avec un embout buccal (20) ayant une lumière qui a une surface de section transversale inférieure ou égale à 80 mm², éventuellement inférieure ou égale à 50 mm² et/ou dans lequel le premier moyen d'engagement d'embout buccal (12) est adapté pour s'engager avec un embout buccal ayant une lumière qui a une surface de section transversale supérieure ou égale à 380 mm², éventuellement supérieure ou égale à 700 mm².

6. Dispositif capteur de gaz comprenant une interface (10) tel que défini dans l'une quelconque des revendications 1 à 6, comprenant en outre un embout buccal fixé au premier (12) ou deuxième (14) moyen d'engagement d'embout buccal, et comprenant de préférence un embout buccal (20), ayant une section transversale essentiellement circulaire et ayant un diamètre inférieur ou égal à 10 mm, fixé au deuxième moyen d'engagement d'embout buccal (14).

7. Dispositif capteur de gaz selon la revendication 6, qui comprend un embout buccal comprenant un repère, le repère indique la distance correcte pour l'insertion de l'embout buccal dans la bouche d'un utilisateur.

8. Procédé de fonctionnement du dispositif capteur de gaz comprenant les étapes qui consistent :
(1) à fournir un dispositif capteur de gaz selon l'une quelconque des revendications 1 à 7,
(2) à fournir un embout buccal approprié (20), et
(3) à fixer l'embout buccal au dispositif capteur de gaz, et de préférence
(4) à amener un sujet à tester à expirer dans le dispositif de détection de gaz à travers l'embout buccal (20), où l'embout buccal (20) présente de préférence une surface de section transversale inférieure ou égale à 180 mm², plus préférablement inférieure ou égale à 80 mm², éventuellement inférieure ou égale à 50 mm².

9. Kit comprenant un dispositif capteur de gaz de l'une quelconque des revendications 1 à 7 avec un embout buccal ou plusieurs embouts buccaux (20).
